# EUROPEAN PATENT SPECIFICATION

(11) **EP 0 778 262 B1**
(45) Date of publication and mention of the grant of the patent: **24.01.2001**
(21) Application number: 96308347.2
(22) Date of filing: 19.11.1996
(51) Int. Cl.: C07C 213/10, C07C 217/74

(54) **Process for the purification of (RR-SS)-2-dimethyl-aminomethyl-1-(3-methoxyphenyl)cyclohexanol and its salts**
Verfahren zur Reinigung von (RR-SS)-2-Dimethyl-Aminomethyl-1-(3-Methoxyphenyl)Cyclohexanol und dessen Salzen
Procédé pour la purification du (RR-SS)-2-diméthyl-aminométhyl-1-(3-méthoxyphényl)cyclohexanol et ses sels

(30) Priority: 07.12.1995 IL 11628195
(43) Date of publication of application: 11.06.1997
(73) Proprietor: Chemagis Ltd., Bnei Brak (IL)
(72) Inventor: Lerman, Ori, Ramat Gan 52501 (IL); Tennenbaum, Michael, Rosh Haayin 48590 (IL); Gal, Erez, Givataim 53444 (IL); Kaspi, Joseph, Givataim 53201 (IL)
(74) Representative: Ablewhite, Alan James

(56) References cited:
- US-A- 3 652 589
- US-A- 5 414 129

## Description

The present invention relates to a process for the purification of (RR,SS)-2-dimethylaminomethyl-1-(3-methoxyphenyl)cyclohexanol hydrochloride, also known as tramadol, from (RS,SR)-2-dimethylaminomethyl-1-(3-methoxyphenyl)cyclohexanol hydrochloride and other undesirable products. More particularly the present invention is based on the newly discovered observation that (RS,SR)-2-dimethylaminomethyl-1-(3-methoxyphenyl)cyclohexanol undergoes dehydration under certain conditions, while (RR,SS)-2-dimethylaminomethyl-1-(3-methoxyphenyl)cyclohexanol remains practically intact.

Tramadol is a well-established pain-killer invented by Gruenenthal GmbH, Germany, used as a non-addictive analgestic and sold under different trade names such as Tramal, Crispin or Tramundin.

The synthesis of tramadol is described in US Pat. No. 3652589 and in British Pat. No. 992399.

The standard commercial synthesis of tramadol consists of a Grignard reaction between 2-dimethylaminomethylcyclohexanone and 3-methoxyphenylmagnesium bromide (Equation 1).

From the reaction nature, it is clear that both (RR,SS)-2-dimethylaminomethyl-1-(3-methoxyphenyl)cyclohexanol (Structure 1) and (RS,SR)-2-dimethylaminomethyl-1-(3-methoxyphenyl)cyclohexanol (Structure 2) are obtained in variable ratios, depending on the reaction conditions.

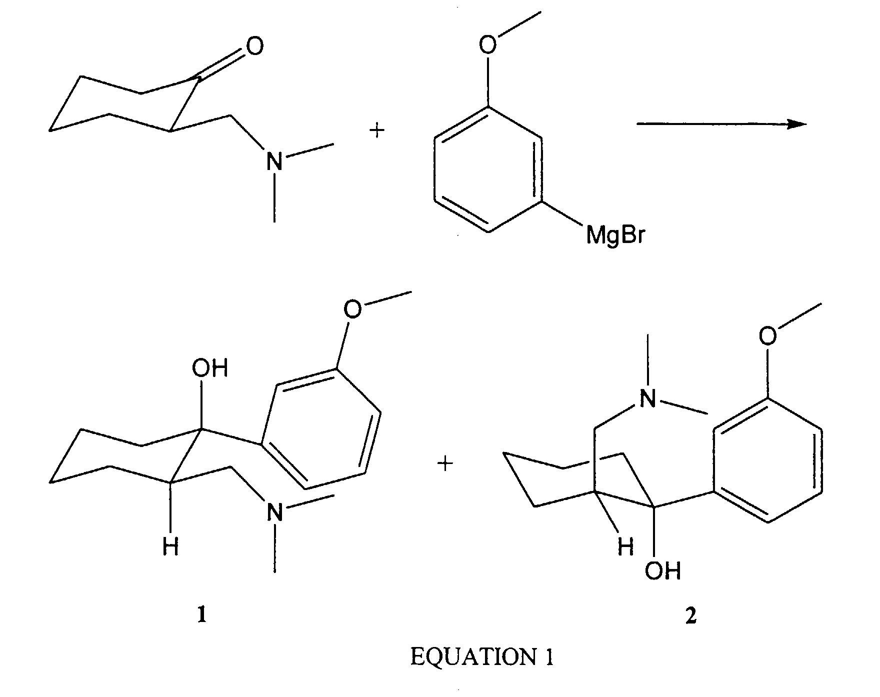

The original patents assigned to Gruenenthal GmbH describe the isolation of pure -(RR,SS)-2-dimethylaminomethyl-1-(3-methoxyphenyl)cyclohexanol as follows: The complex mixture of products containing (RR,SS)- and (RS,SR)-2-dimethylaminomethyl-1-(3-methoxyphenyl)cyclohexanol obtained from the Grignard reaction is distilled under high vacuum. Both isomers distill at 138°-140°C, (0.8 mbar [0.6 mm Hg]). The distillate is dissolved in ether, and the solution is treated with gaseous HC 1. The resulting mixture of (RR,SS)- and (RS, SR)-2-dimethylaminomethyl-1-(3-methoxyphenyl)cyclohexanol hydrochloride is precipitated and filtered, usually this mixture contains about 20% of (RS,SR)-2-dimethylaminomethyl-1-(3-methoxyphenyl)cyclohexanol hydrochloride.

The mixture of isomers is then refluxed twice with five volumes of moist dioxane, and filtered. The cake obtained consists of pure (RR,SS)-2-dimethylaminomethyl-1-(3-methoxyphenyl)cyclohexanol hydrochloride, while the residual solution consists of "a mixture of about 20% to 30% of the cis (i.e. RS,SR) isomer and about 70% to 80% of the trans (i.e. RR,SS) isomer which cannot be further separated by boiling dioxane" (US 3652589 - Example 2).

Dioxane, used in large quantities in this process, possesses many undesirable properties. It has recently been listed as Category 1 carcinogen by OSHA. (Kirk & Othmer, 3rd edition, vol. 9, page 386) and it is known to cause CNS depression and liver necrosis (ibid. Vol. 13, page 267). In addition, it tends to form hazardous peroxides (ibid. Vol. 17, page 48).

As a result, the concentration of dioxane in the final product has been strictly limited to several ppb's, and the DAC (1991) restricted the level of dioxane in tramadol to 500 ppb.

Another separation method patented by Chemagis Ltd. (IL 103096) takes advantage of the fact that the precipitation of (RR,SS)-2-dimethylaminomethyl-1-(3-methoxyphenyl)cyclohexanol hydrochloride from its solution in medium chained alcohols (C₄-C₈) occurs faster than the precipitation of (RS,SR)-2-dimethylaminomethyl-1-(3-methoxyphenyl)cyclohexanol hydrochloride which tends to separate later.

The main disadvantage of this method is that the time interval between the end of separation of (RR,SS)-2-dimethylaminomethyl-1-(3-methoxyphenyl)cyclohexanol hydrochloride and the start of the (RS,SR)-2-dimethylaminomethyl-1-(3-methoxyphenyl)cyclohexanol hydrochloride separation is variable, and seems to depend sharply on the composition of the crude mixture. Therefore, variations in the yield and the quality of the product often occur. Furthermore, about 40% of (RR,SS)-2-dimethylaminomethyl-1-(3-methoxyphenyl)cyclohexanol hydrochloride does not separate and remains in solution, along with (RS,SR)-2-dimethylaminomethyl-1-(3-methoxyphenyl)cyclohexanol hydrochloride. This mixture cannot be further purified by this method.

After performing a series of experiments in order to investigate the chemical properties of (RS,SR)- and (RR,SS)-2-dimethylaminomethyl-1-(3-methoxyphenyl)cyclohexanol, it has now been surprisingly found, that the isomers exhibit considerable differences in their chemical behaviour, namely (RS,SR)-2-dimethylaminomethyl-1-(3-methoxyphenyl)cyclohexanol has been found to be considerably more reactive towards dehydration than (RR,SS)-2-dimethylaminomethyl-1-(3-methoxyphenyl)cyclohexanol. Practically, when a mixture of (RR,SS)- and (RS, SR)-2-dimethylaminomethyl-1-(3-methoxyphenyl)cyclohexanol is heated with a strong acid in an organic or aqueous medium, (RS,SR)-2-dimethylaminomethyl-1-(3-methoxyphenyl)cyclohexanol undergoes selective dehydration, while (RR,SS)-2-dimethylaminomethyl-1-(3-methoxyphenyl)cyclohexanol remains intact. The main dehydration product is 1-(-3-methoxyphenyl)-2-dimethylaminomethyl - cyclohex-6-ene (**3**), while minor quantities of 1-(3-methoxyphenyl)-2-dimethylaminomethyl cyclohex-1-ene.

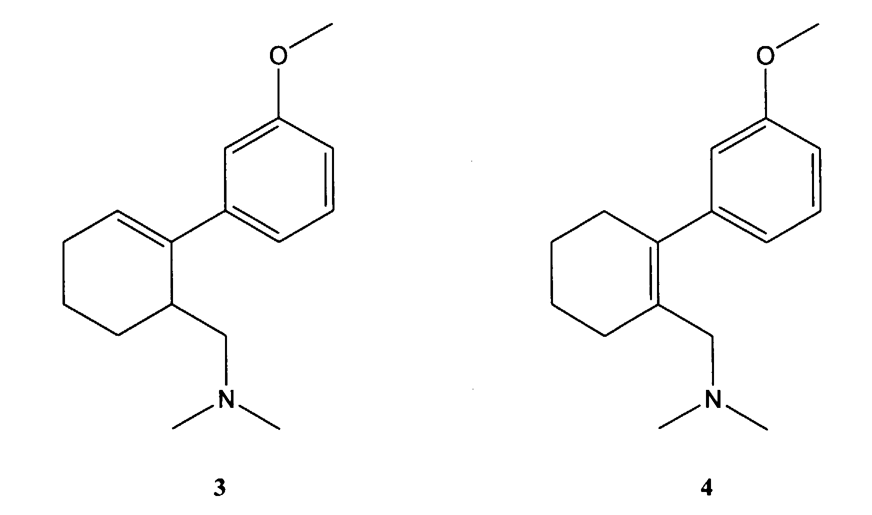

Once most of the (RS,SR)-2-dimethylaminomethyl-1-(3-methoxyphenyl)cyclohexanol is converted to the dehydrated compounds. the resulting (RR,SS)-2-dimethylaminomethyl-1-(3-methoxyphenyl)cyclohexanol can be easily purified from the other compounds by simple recrystallization.

In retrospect. looking at the structure of (RS,SR)- and (RR,SS)-2-dimethylaminomethyl-1-(3-methoxyphenyl)cyclohexanol, it is evident why (RS,SR)-2-dimethylaminomethyl-1-(3-methoxyphenyl)cyclohexanol is more susceptible to dehydration.

The proton attached to the nitrogen of protonated (RR,SS)-2-dimethylaminomethyl-1-(3-methoxyphenyl)cyclohexanol is capable of forming a stable hydrogen bond with the oxygen atom of the hydroxyl group, (see Fig. 2). In (RS,SR)-2-dimethylaminomethyl-1-(3-methoxyphenyl)cyclohexanol, there is no possible way of forming a stable intramolecular hydrogen bond, (see Fig. 1).

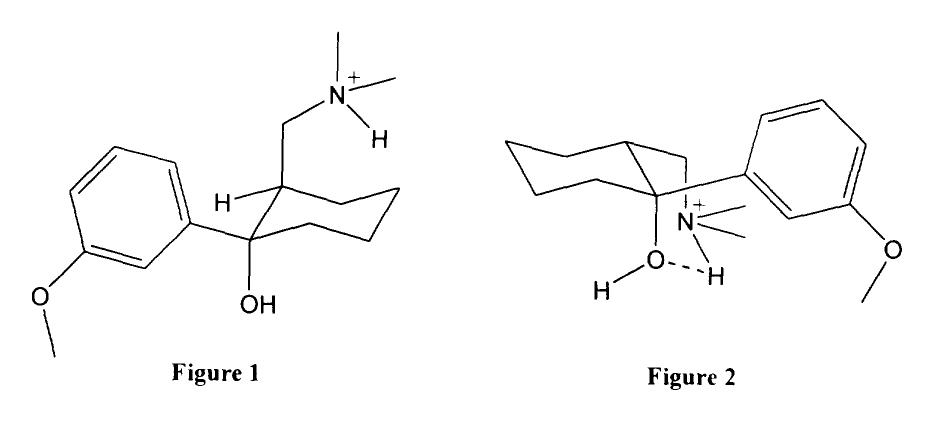

Furthermore. when the reaction is carried out in aqueous medium. a certain amount of (RS,SR)-2-dimethylaminomethyl-1-(3-methoxyphenyl)cyclohexanol. up to 50%, is converted into (RR,SS)-2-dimethylaminomethyl-1-(3-methoxyphenyl)cyclohexanol. This may, of course, increase the efficiency of the process.

Thus, according to the present invention there is now provided a process for the purification and isolation of (RR,SS)-2-dimethylaminomethyl-1-(3-methoxyphenyl)cyclohexanol from mixtures also containing (RS,SR)-2-dimethylaminomethyl-1-(3-methoxyphenyl)cyclohexanol comprising:
a. reacting the above mixture with a strong acid in an organic or aqueous medium at elevated temperature, whereby (RS,SR)-2-dimethylaminomethyl-1-(3-methoxyphenyl)cyclohexanol is selectively converted into, 1-(3-methoxyphenyl)-2-dimethylaminomethylcyclohex-6-ene, 1 -(3 -methoxyphenyl)-2-dimethyl-aminomethylcyclohex-1-ene or a mixture thereof; or, when the reaction is carried out in aqueous medium, 1-(3-methoxyphenyl)-2-dimethylaminomethylcyclohex-6-ene, 1-(3-methoxyphenyl)-2-dimethylaminomethylcyclohex-1-ene, (RR,SS)-2-dimethylaminomethyl-1-(3-methoxyphenyl)cyclohexanol, or a mixture thereof;
b. selectively precipitating the desired (RR,SS)-2-dimethylaminomethyl-1-(3-methoxyphenyl)cyclohexanol as an amine acid salt; and
c. recrystallizing the purified product.

Treatment with aqueous acidic solution can be applied in order to purify mixtures of of (RS,SR)- and (RR,SS)-2-dimethylaminomethyl-1-(3-methoxyphenyl)cyclohexanol hydrochloride, such as the residues obtained from the purification process using dioxane, as described in US Patent 3653589, or from the purification process with medium chained alcohols, as described in Israeli Patent 103096. Furthermore, pure (RS,SR)-2-dimethylaminomethyl-1-(3-methoxyphenyl)cyclohexanol can be partially converted to the desired (RR,SS)-2-dimethylaminomethyl-1-(3-methoxyphenyl)cyclohexanol when treated under the above-mentioned conditions.

The general purification procedure consists of heating the mixture of (RR,SS)-2-dimethylaminomethyl-1-(3-methoxyphenyl)cyclohexanol and (RS,SR)-2-dimethylaminomethyl-1-(3-methoxyphenyl)cyclohexanol obtained from the Grignard reaction between 2-dimethylaminomethyl cyclohexanone and 3-methoxyphenylmagnesium-bromide with strong acid in aqueous medium. The resulting mixture is brought to pH 13, extracted with toluene and the organic phase is evaporated under reduced pressure. The resulting oil is treated with HCl solution in 2-propanol, and cooled to 5°C. (RR,SS)-2-dimethylaminomethyl-1-(3-methoxyphenyl)cyclohexanol hydrochloride separates exclusively, while most of the other ammonium salts [including (RS,SR)-2-dimethylaminomethyl-1-(3-methoxyphenyl)cyclohexanol hydrochloride, and the hydrochlorides of the dehydrated derivatives of 2-dimethylaminomethyl-1-(3-methoxyphenyl)cyclohexanol stay in solution. The crude (RR,SS)-2-dimethylaminomethyl-1-(3-methoxyphenyl)cyclohexanol hydrochloride obtained can be purified by recrystallization.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### EXAMPLE 1

923 g of the solution obtained from Grignard reaction between 2-dimethylaminomethylcyclohexanone and 3-methoxyphenylmagnesium bromide is evaporated under reduced pressure until no solvent distillation is observed. 212 g of thick oil containing 43.5% of (RR,SS)-2-dimethylaminomethyl-1-(3-methoxyphenyl)cyclohexanol and 8% of (RS,SR)-2-dimethylaminomethyl-1-(3-methoxyphenyl)cyclohexanol is obtained. The isomer ratio is about 1:5.5. 530 g of water and 180 g of 4-toluenesulfonic acid monohydrate are added and the mixture is heated to 100°C for 2 hours. The mixture is cooled and brought to pH 8, 100 g of solid potassium carbonate and 250 ml of toluene are added thereinto. and the phases are separated.

The organic phase is evaporated and 173 g of dark oil containing 48.7% of (RR,SS)-2-dimethylaminomethyl-1-(3-methoxyphenyl)cyclohexanol and 2.5% of (RS,SR)-2-dimethylaminomethyl-1-(3-methoxyphenyl)cyclohexanol is obtained. The ratio of (RS.SR)-2-dimethylaminomethyl-1-(3-methoxyphenyl)cyclohexanol to (RR,SS)-2-dimethylaminomethyl-1-(3-methoxyphenyl)cyclohexanol is 1:20 and the yield of (RR,SS)-2-dimethylaminomethyl-1-(3-methoxyphenyl)cyclohexanol is 92% based on the starting amount of (RR,SS)-2-dimethylaminomethyl-1-(3-methoxyphenyl)cyclohexanol.

### EXAMPLE 2

172 g of the crude product obtained from Example 1 was dissolved in 150 ml of 2-propanol. 110 ml of 23% solution of the HCl in 2-propanol is gradually added while the temperature is kept below 25 ° C. The mixture is then stirred and cooled to 5 °C for 10 hours. (RR.SS)-2-dimethylaminomethyl-1-(3-methoxyphenyl)cyclohexanol hydrochloride is separated almost in pure form. The crude product is recrystallized from 2-propanol.

### EXAMPLE 3

1000 g of the mixture obtained from Grignard reaction between 2-diethylaminomethyl cyclohexanone and 3-methoxyphenylmagnesium bromide is evaporated under reduced pressure until no solvent distillation is observed. 225 g of dark oil containing 43.5% of (RR.SS)-2-dimethylaminomethyl-1-(3-methoxyphenyl)cyclohexanol and 8% of (RS.SR)-2-dimethylaminomethyl-1-(3-methoxyphenyl)cyclohexanol is obtained. The ratio of (RS,SR)-2-dimethylaminomethyl-1-(3-methoxyphenyl)cyclohexanol to (RR,SS)-2-dimethylaminomethyl-1-(3-methoxyphenyl)cyclohexanol is 1:5.5. 60 ml of toluene and 18 g of 4-toluenesulfonic acid monohydrate are added and the mixture is heated to 90°C for 2 hours. The reaction is worked up in the same manner as described in Example 1. The ratio of (RS,SR)-2-dimethylaminomethyl-1-(3-methoxyphenyl)cyclohexanol to (RR,SS)-2-dimethylaminomethyl-1-(3-methoxyphenyl)cyclohexanol is 1:8 and the yield of (RR,SS)-2-dimethylaminomethyl-1-(3-methoxyphenyl)cyclohexanol is 95% based on the starting amount of (RR,SS)-2-dimethylaminomethyl-1-(3-methoxyphenyl)cyclohexanol.

### EXAMPLE 4

The solution obtained after the Grignard reaction is treated with 4-toluenesulfonic acid in the same manner as described in Example 1, but the reaction mixture is heated to 90°C for 2-1/2 hours. At the end of the reaction the ratio of (RS,SR)-2-dimethylaminomethyl-1-(3-methoxyphenyl)cyclohexanol to (RR,SS)-2-dimethylaminomethyl-1-(3-methoxyphenyl)cyclohexanol is 1:18 and the yield of (RR.SS)-2-dimethylaminomethyl-1-(3-methoxyphenyl)cyclohexanol is 102% based on the starting amount of (RR.SS)-2-dimethylaminomethyl-1-(3-methoxyphenyl)cyclohexanol. The product is worked up as described in Example 2 to give the final product.

### EXAMPLE 5

The solution obtained after the Grignard reaction is treated with 4-toluenesulfonic acid in the same manner as described in Example 1, but the reaction mixture is heated to 75 ° C and the reaction is stopped after 4 hours. At the end of the reaction the ratio of (RS,SR)-2-dimethylaminomethyl-1-(3-methoxyphenyl)cyclohexanol to (RR,SS)-2-dimethylaminomethyl-1-(3-methoxyphenyl)cyclohexanol was 1:8 and the yield of (RR,SS)-2-dimethylaminomethyl-1-(3-methoxyphenyl)cyclohexanol is 99% based on the starting amount of (RR,SS)-2-dimethylaminomethyl-1-(3-methoxyphenyl)cyclohexanol.

### EXAMPLE 6

To 8.8 g of pure (RS.SR)-2-dimethylaminomethyl-1-(3-methoxyphenyl)cyclohexanol are added 25 ml of water and 10 g of 4-toluenesulfonic acid monohydrate. The mixture is heated to 95 ° C for 2 hours. The reaction is stopped and worked up in the same manner as described in Example 1. 8 g of oil consisting of 52.8% of (RR,SS)-2-dimethylaminomethyl-1-(3-methoxyphenyl)cyclohexanol, 5% of (RS,SR)-2-dimethylaminomethyl-1-(3-methoxyphenyl)cyclohexanol and 37.8% of 2-dimethylaminomethyl-1-(3-methoxyphenyl) cyclohex-6-ene are obtained.

### EXAMPLE 7

3600 ml of filtrate containing 7% of (RR,SS)-2-dimethylaminomethyl-1-(3-methoxyphenyl)cyclohexanol hydrochloride and 1.7% of (RS,SR)-2-dimethylaminomethyl-1-(3-methoxyphenyl)cyclohexanol hydrochloride dissolved in a mixture of primary alcohols obtained as mother liquor from the purifying process for Tramadol as described in Israeli Pat. 103096 is evaporated to about one half of its volume, and the dark viscous solution obtained is extracted with 2 x 1500 ml of water.

The aqueous phase is brought to pH 7 with 40% NaOH solution then 500 g of solid potassium carbonate and 1500 ml of toluene are added.

The mixture is stirred. the layers separated, and the organic layer was evaporated to dryness.

230 g 4-toluenesulfonic acid monohydrate and 456 ml of water are added to the residue, and the mixture is heated to 90°C for 2-1/2 hours. The reaction is stopped and worked up in the same manner as described in Example 1. The ratio of (RS,SR)-2-dimethylaminomethyl-1-(3-methoxyphenyl)cyclohexanol to (RR,SS)-2-dimethylaminomethyl-1-(3-methoxyphenyl)cyclohexanol is 1:20, and the yield up to this point is 76% based on the starting amount of (RR,SS)-2-dimethylaminomethyl-1-(3-methoxyphenyl)cyclohexanol hydrochloride. Pure (RR,SS)-2-dimethylaminomethyl-1-(3-methoxyphenyl)cyclohexanol hydrochloride was resolved from the dark oil as described in Example 2.

### EXAMPLE 8

71 g of isomers mixture containing 51 g of (RR.SS)-2-dimethylaminomethyl-1-(3-methoxyphenyl)cyclohexanol hydrochloride and 20 g of (RS,SR)-2-dimethylaminomethyl-1-(3-methoxyphenyl)cyclohexanol hydrochloride obtained as a result of the purification method described in US Patent 3652589 was converted to the corresponding base mixture by treating with 30% K₂CO₃ mixture. 62 g of mixture is obtained.

The mixture is heated to 90°C with 90 g 4-toluenesulfonic acid and 250 ml H₂O for 2 1/2 hours and the mixture was worked up as described in Example 2.

The ratio of (RS,SR)-2-dimethylaminomethyl-1-(3-methoxyphenyl)cyclohexanol to (RR,SS)-2-dimethylaminomethyl-1-(3-methoxyphenyl)cyclohexanol is 1:20 and the yield of (RR,SS)-2-dimethylaminomethyl-1-(3-methoxyphenyl)cyclohexanol is 111% based on the starting amount of (RR,SS)-2-dimethylaminomethyl-1-(3-methoxyphenyl)cyclohexanol hydrochloride.

### EXAMPLE 9

A mixture prepared from 5 g (RR,SS)-2-dimethylaminomethyl-1-(3-methoxyphenyl)cyclohexanol and 5 g (RS.SR)-2-dimethylaminomethyl-1-(3-methoxyphenyl)cyclohexanol is heated for 3 hours to 90°C with 50 ml of water and 15 g of 4-toluenesulfonic acid monohydrate for 2 hours. The reaction is stopped and worked up in the same manner as described in Example 1.

The ratio of (RS,SR)-2-dimethylaminomethyl-1-(3-methoxyphenyl)cyclohexanol to (RR.SS)-2-dimethylaminomethyl-1-(3-methoxyphenyl)cyclohexanol is 1:18 and the yield of (RR,SS)-2-dimethylaminomethyl-1-(3-methoxyphenyl)cyclohexanol is 122% based on the starting amount of (RR.SS)-2-dimethylaminomethyl-1-(3-methoxyphenyl)cyclohexanol.

### EXAMPLE 10

923 g of the solution obtained from Grignard reaction between 2-dimethylaminomethylcyclohexanone and 3-methoxyphenylmagnesium bromide is evaporated under reduced pressure until no solvent distillation is observed. 215 g of thick oil is obtained. 250 ml of 25% aqueous solution of sulfuric acid is introduced thereinto. and the mixture is heated to 90°C for 2 hours. The mixture is brought to pH 11 with solid potassium carbonate and extracted with toluene.

The ratio of (RS,SR)-2-dimethylaminomethyl-1-(3-methoxyphenyl)cyclohexanol to (RR.SS)-2-dimethylaminomethyl-1-(3-methoxyphenyl)cyclohexanol is 1:18 and yield of (RR.SS)-2-dimethylaminomethyl-1-(3-methoxyphenyl)cyclohexanol is 120% based on the starting amount of (RR,SS)-2-dimethylaminomethyl-1-(3-methoxyphenyl)cyclohexanol.

### EXAMPLE 11

115 g of thick oil are obtained from Grignard reaction between 2-dimethylaminomethylcyclohexanone and 3-methoxyphenylmagnesium bromide, containing (RR,SS)-2-dimethylaminomethyl-1-(3-methoxyphenyl)cyclohexanol and (RS.SR)-2-dimethylaminomethyl-1-(3-methoxyphenyl)cyclohexanol in the ratio of 1:5.5. The oil is heated to 90°C with 250 ml of 50% solution of phosphoric acid for 2.5 hours. The dark oil resulted contains (RS,SR)-2-dimethylaminomethyl-1-(3-methoxyphenyl)cyclohexanol and (RR.SS)-2-dimethylaminomethyl-1-(3-methoxyphenyl)cyclohexanol in the ratio of 1:18. The yield of (RR,SS)-2-dimethylaminomethyl-1-(3-methoxyphenyl)cyclohexanol is 75% based on the starting amount of (RR,SS)-2-dimethylaminomethyl-1-(3-methoxyphenyl)cyclohexanol.

### EXAMPLE 12

115 g of thick oil are obtained from Grignard reaction between 2-dimethylaminomethylcyclohexanone and 3-methoxyphenylmagnesium-bromide containing (RR,SS)-2-dimethylaminomethyl-1-(3-methoxyphenyl)cyclohexanol and (RS,SR)-2-dimethylaminomethyl-1-(3-methoxyphenyl)cyclohexanol in the ratio of 1:5.5. The oil is heated to 90°C with 250 ml of 70% solution of formic acid for 2.5 hours. The dark oil resulting contains (RS,SR)-2-dimethylaminomethyl-1-(3-methoxyphenyl)cyclohexanol and (RR,SS)-2-dimethylaminomethyl-1-(3-methoxyphenyl)cyclohexanol. in the ratio of 1:9. The yield of (RR,SS)-2-dimethylaminomethyl-1-(3-methoxyphenyl)cyclohexanol is 66% based on the starting amount of (RR,SS)-2-dimethylaminomethyl-1-(3-methoxyphenyl)cyclohexanol.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims. rather than to the foregoing description, and all changes which come within the meaning and range of equivalency of the claims are therefore intended to be embraced therein.

## Claims

1. A process for the purification and isolation of (RR,SS)-2-dimethylaminomethyl-1-(3-methoxyphenyl)cyclohexanol from mixtures also containing (RS,SR)-2-dimethylaminomethyl-1-(3-methoxyphenyl)cyclohexanol comprising:
a. reacting the above mixture with a strong acid in an organic or aqueous medium at elevated temperature, whereby (RS,SR)-2-dimethylaminomethyl-1-(3-methoxyphenyl)cyclohexanol is selectively converted into 1-(3-methoxyphenyl)-2-dimethylaminomethylcyclohex-6-ene, 1-(3-methoxyphenyl)-2-dimethylaminomethylcyclohex-1-ene or a mixture thereof; or, when the reaction is carried out in aqueous medium, 1-(3-methoxyphenyl)-2-dimethylaminomethylcyclohex-6-ene, 1-(3-methoxyphenyl)-2-dimethylaminomethylcyclohex-1-ene, (RR,SS)-2-dimethylaminomethyl-1-(3-methoxyphenyl)cyclohexanol, or a mixture thereof;
b. selectively precipitating the desired (RR.SS)-2-dimethylaminomethyl-1-(3-methoxyphenyl)cyclohexanol as an amine acid salt; and
c. recrystallizing the purified product.

2. A process according to Claim 1 wherein the mixture of (RR,SS)-2-dimethylaminomethyl-1-(3-methoxyphenyl)cyclohexanol and (RS,SR)-2-dimethylaminomethyl-1-(3-methoxyphenyl)cyclohexanol is obtained by the reaction of 2-dimethylaminomethylcyclohexanone and 3-methoxyphenylmagnesium bromide.

3. A process according to Claim 1 or Claim 2, wherein said mixture of (RR,SS)-2-dimethylaminomethyl-1-(3-methoxyphenyl)cyclohexanol and (RS,SR)-2-dimethylaminomethyl-1-(3-methoxyphenyl)cyclohexanol comprises from 20% to 30% (RS,SR)-2-dimethylaminomethyl-1-(3-methoxyphenyl)cyclohexanol, and from 70% to 80% (RR,SS)-2-dimethylaminomethyl-1-(3-methoxyphenyl)cyclohexanol.

4. A process according to Claim 1 or Claim 2, wherein said mixture of (RR,SS)-2-dimethylaminomethyl-1-(3-methoxyphenyl)cyclohexanol and (RS,SR)-2-dimethylaminomethyl-1-(3-methoxyphenyl)cyclohexanol is obtained from a solution of the corresponding hydrochlorides in C₄-C₈ alcohols from which (RR,SS)-2-dimethylaminomethyl-1-(3-methoxyphenyl)cyclohexanol hydrochloride has been precipitated.

5. A process according to Claim 1, wherein the reacting substance is pure (RS,SR)-2-dimethylaminomethyl-1-(3-methoxyphenyl) cyclohexanol.

6. A process according to any of Claims 1 to 5, wherein the reaction is carried out at a temperature between 50°-120°C.

7. A process according to any of claims 1 to 6, wherein the reaction is carried out at boiling point.

8. A process according to any of Claims 1 to 7, wherein the reaction is carried out in aqueous medium.

9. A process according to any of Claims 1 to 8, wherein said strong acid is sulfuric acid.

10. A process according to any of Claims 1 to 8, wherein said strong acid is 4-toluenesulfonic acid.

11. A process according to any of Claims 1 to 10, wherein the precipitation of Step b is carried out using an alcohol and hydrogen chloride.

12. A process according to any of Claims 1 to 11, wherein the final precipitation is carried out by recrystallization from an alcohol.

13. A process according to Claim 11, wherein the alcohol is 2-propanol.

## Patentansprüche

1. Verfahren zur Reinigung und Isolierung von (RR,SS)-2-Dimethylaminomethyl-1-(3-methoxyphenyl)cyclohexanol aus Mischungen die ebenfalls (RS,SR)-2-Dimethylaminomethyl-1-(3-methoxyphenyl)cyclohexanol enthalten, Verfahren mit folgenden Verfahrensschritten :
a. Reaktion der obigen Mischung mit einer starken Säure in einem organischen oder wässrigen Medium bei hohen Temperaturen, wodurch (RS,SR)-2-Dimethylaminomethyl-1-(3-methoxyphenyl)cyclohexanol selektiv umgewandelt wird in 1-(3-Methoxyphenyl)-2-dimethylaminomethylcyclohex-6-en, 1-(3-Methoxyphenyl)-2-dimethylaminomethylcyclohex-1-en oder eine Mischung derselben; oder, wenn die Reaktion in einem wässrigen Medium durchgeführt wird, 1-(3-Methoxyphenyl)-2-dimethylaminomethylcyclohex-6-en, 1-(3-Methoxyphenyl)-2-dimethylaminomethylcyclohex-1-en, (RR,SS)-2-Dimethylaminomethyl-1-(3-methoxyphenyl)cyclohexanol, oder eine Mischung derselben;
b. selektive Fällung des gewünschten (RR,SS)-2-Dimethylaminomethyl-1-(3-methoxyphenyl)cyclohexanols als ein Aminosäuresalz; und
c. Umkristallisierung des gereinigten Produktes.

2. Verfahren gemäß Anspruch 1, bei welchem die Mischung aus (RR,SS)-2-Dimethylaminomethyl-1-(3-methoxyphenyl)cyclohexanol und (RS,SR)-2-Dimethylaminomethyl-1-(3-methoxyphenyl)cyclohexanol erzielt wird durch die Reaktion von 2-Dimethylaminomethylcyclohexanon und 3-Methoxyphenylmagnesiumbromid.

3. Verfahren gemäß Anspruch 1 oder Anspruch 2, bei welchem die Mischung aus (RR,SS)-2-Dimethylaminomethyl-1-(3-methoxyphenyl)cyclohexanol und (RS,SR)-2-Dimethylaminomethyl-1-(3-methoxyphenyl)cyclohexanol zwischen 20% und 30% (RS,SR)-2-Dimethylaminomethyl-1-(3-methoxyphenyl)cyclohexanol, sowie zwischen 70% und 80% (RR,SS)-2-Dimethylaminomethyl-1-(3-methoxyphenyl)cyclohexanol enthält.

4. Verfahren gemäß Anspruch 1 oder Anspruch 2, bei welchem die Mischung aus (RR,SS)-2-Dimethylaminomethyl-1-(3-methoxyphenyl)cyclohexanol und (RS,SR)-2-Dimethylaminomethyl-1-(3-methoxyphenyl)cyclohexanol erzielt wird aus einer Lösung des entsprechenden Hydrochlorids in C₄-C₈ Alkoholen aus welchen (RR,SS)-2-Dimethylaminomethyl-1-(3-methoxyphenyl)cyclohexanol-hydrochlorid ausgefällt worden ist.

5. Verfahren gemäß Anspruch 1, bei welchem die reagierende Substanz reines (RS,SR)-2-Dimethylaminomethyl-1-(3-methoxyphenyl)cyclohexanol ist.

6. Verfahren gemäß irgendeinem der Ansprüche 1 bis 5, bei welchem die Reaktion bei einer Temperatur zwischen 50° und 120°C durchgeführt wird.

7. Verfahren gemäß irgendeinem der Ansprüche 1 bis 6, bei welchem die Reaktion bei der Siedetemperatur durchgeführt wird.

8. Verfahren gemäß irgendeinem der Ansprüche 1 bis 7, bei welchem die Reaktion in einem wässrigen Medium durchgeführt wird.

9. Verfahren gemäß irgendeinem der Ansprüche 1 bis 8, bei welchem die starke Säure Schwefelsäure ist.

10. Verfahren gemäß irgendeinem der Ansprüche 1 bis 8, bei welchem die starke Säure 4-Toluolsulfonsäure ist.

11. Verfahren gemäß irgendeinem der Ansprüche 1 bis 10, bei welchem die Fällung gemäß Schritt b unter Einsatz von Alkohol und Chlorwasserstoff durchgeführt wird.

12. Verfahren gemäß irgendeinem der Ansprüche 1 bis 11, bei welchem die endgültige Fällung durch Umkristallisierung aus Alkohol durchgeführt wird.

13. Verfahren gemäß Anspruch 11, in welchem der Alkohol Propanol-2 ist.

## Revendications

1. Procédé pour la purification et l'isolement de (RR,SS)-2-diméthylaminométhyl-1-(3-méthoxyphényl)cyclohexanol à partir de mélanges contenant également du (RS,SR)-2-diméthylaminométhyl-1-(3-méthoxyphényl)cyclohexanol comprenant:
a. la réaction du mélange ci-dessus avec un acide fort dans un milieu organique ou aqueux à une température élevée, ce par quoi le (RS,SR)-2-diméthylaminométhyl-1-(3-méthoxyphényl)cyclohexanol est sélectivement converti en 1-(3-méthoxyphényl)-2-diméthylaminométhylcyclohex-6-ène, 1-(3-méthoxyphényl)-2-diméthylaminométhylcyclohex-1-ène ou un de leurs mélanges; ou, lorsque la réaction est effectuée en milieu aqueux, 1-(3-méthoxyphényl)-2-diméthylaminométhylcyclohex-6-ène, 1 -(3-méthoxyphényl)-2-diméthylaminométhylcyclohex-1-ène, (RR,SS)-2-diméthylaminométhyl-1-(3-méthoxyphényl)cyclohexanol, ou un de leurs mélanges;
b. la précipitation sélective du (RR,SS)-2-diméthylaminométhyl-1-(3-méthoxyphényl)cyclohexanol désiré sous la forme d'un sel acide d'amine; et
c. la recristallisation du produit purifié.

2. Procédé selon la revendication 1 dans lequel le mélange de (RR,SS)-2-diméthylaminométhyl-1-(3-méthoxyphényl)cyclohexanol et de (RS,SR)-2-diméthylaminométhyl-l-(3-méthoxyphényl)cyclohexanol est obtenu par la réaction de la 2-diméthylaminométhylcyclohexanone et du bromure de 3-méthoxyphénylmagnésium.

3. Procédé selon la revendication 1 ou la revendication 2, dans lequel ledit mélange de (RR,SS)-2-diméthylaminométhyl-1-(3-méthoxyphényl)cyclohexanol et de (RS,SR)-2-diméthylaminométhyl-1-(3-méthoxyphényl)cyclohexanol comprend 20% à 30% de (RS,SR)-2-diméthylaminométhyl-1-(3-méthoxyphényl)cyclohexanol, et 70% à 80% de (RR,SS)-2-diméthylaminométhyl-1-(3-méthoxyphényl)cyclohexanol.

4. Procédé selon la revendication 1 ou la revendication 2, dans lequel ledit mélange de (RR,SS)-2-diméthylaminométhyl-1-(3-méthoxyphényl)cyclohexanol et de (RS,SR)-2-diméthylaminométhyl-1-(3-méthoxyphényl)cyclohexanol est obtenu à partir d'une solution des chlorhydrates correspondants dans des alcools en C₄-C₈ dans laquelle le chlorhydrate de (RR,SS)-2-diméthylaminométhyl-1-(3-méthoxyphényl)cyclohexanol a été précipité.

5. Procédé selon la revendication 1, dans lequel la substance réagissante est le (RS,SR)-2-diméthylaminométhyl-1-(3-méthoxyphényl)cyclohexanol pur.

6. Procédé selon l'une quelconque des revendications 1 à 5, dans lequel la réaction est effectuée à une température comprise entre 50°C et 120°C.

7. Procédé selon l'une quelconque des revendications 1 à 6, dans lequel la réaction est effectuée au point d'ébullition.

8. Procédé selon l'une quelconque des revendications 1 à 7, dans lequel la réaction est effectuée en milieu aqueux.

9. Procédé selon l'une quelconque des revendications 1 à 8, dans lequel ledit acide fort est l'acide sulfurique.

10. Procédé selon l'une quelconque des revendications 1 à 8, dans lequel ledit acide fort est l'acide 4-toluènesulfonique.

11. Procédé selon l'une quelconque des revendications 1 à 10, dans lequel la précipitation de l'étape b est effectuée en utilisant un alcool et du chlorure d'hydrogène.

12. Procédé selon l'une quelconque des revendications 1 à 11, dans lequel la précipitation finale est effectuée par recristallisation dans un alcool.

13. Procédé selon la revendication 11, dans lequel l'alcool est le 2-propanol.
